# FASCICULE DE BREVET EUROPEEN

(11) **EP 0 725 570 B1**
(45) Date de publication et mention de la délivrance du brevet: **08.07.1998**
(21) Numéro de dépôt: 94928435.0
(22) Date de dépôt: 27.09.1994
(51) Int. Cl.: A23K 1/16, A23K 1/18, A61K 9/00, A01N 25/00

(54) **COMPOSITION ET SYSTEME A ADMINISTRATION ORALE POUR ANIMAUX**
ZUSAMMENSETZUNG UND SYSTEM ZUR ORALEN VERABREICHUNG AN TIERE
COMPOSITION AND SYSTEM FOR ORAL ADMINISTRATION TO ANIMALS

(30) Priorité: 27.09.1993 FR 9311449
(43) Date de publication de la demande: 14.08.1996
(73) Titulaire: VIRBAC, 06516 Carros (FR)
(72) Inventeur: DERRIEU, Guy, F-06800 Cagnes-sur-Mer (FR); RAYNIER, Bernard, F-06200 Nice (FR); POUGNAS, Jean-Luc, F-06800 Cagnes-sur-Mer (FR); CASTELLI, Luc, F-06700 Saint-Laurent-du-Var (FR)
(74) Mandataire: Vialle-Presles, Marie José
(86) Numéro de dépôt international: FR9401120
(87) Numéro de publication internationale: WO9508931

(56) Documents cités:
- EP-A- 0 204 596
- EP-A- 0 363 733
- EP-A- 0 438 359
- WO-A-89/12393
- US-A- 5 010 685
- US-A- 5 169 645

## Description

La présente invention est relative à une composition et à un système, destinés à permettre l'administration orale de substances chimiques ou médicamenteuses, telles que vitamines, oligo-éléments, aminoacides, substances nutritionnelles, vaccins, etc..., à des animaux domestiques ou sauvages, tels que mammifères, poissons, crustacés, etc...

La présente invention est également relative au procédé d'obtention desdits systèmes et compositions.

On connaît déjà des systèmes permettant l'administration orale de médicaments à des animaux domestiques, de manière à éviter la voie parentérale, qui nécessite une contention de l'animal. On connaît également des systèmes capables de traiter des animaux élevés de manière extensive ou des animaux sauvages, difficiles ou dangereux à contenir.

De tels systèmes ont fait l'objet de Demandes de Brevet ou de Brevets (Brevet EP 0 240 826, Brevet EP 0 208 528 et Demande de Brevet EP 0 421 863).

L'appât décrit dans le Brevet EP 0 240 826 est obtenu par une première coulée, au fond d'un moule, d'un support comprenant un composé lipidique (ayant un point de fusion entre 20° et 60°C), un composé destiné à stabiliser la forme de l'appât et un composé attractif et appétent pour les animaux, une mise en place de la substance active sur la couche de support solidifiée, puis une deuxième coulée dudit support, de manière à recouvrir complètement ladite substance active.

Ce système a l'inconvénient de présenter une faible résistance mécanique et une fragilité au voisinage de la jonction des deux coulées et le rend inapte aux modes de distribution à grande échelle (largage aérien, par exemple, pour traiter des effectifs importants d'animaux sauvages, sur des territoires importants).

Le Brevet EP 208 528 décrit un appât pour poissons et crustacés constitué essentiellement d'un polymère insoluble dans l'eau ayant un point de fusion inférieur à 110°C (polyamides ou copolymères d'éthylène, EVA, en particulier), d'une substance attractive et d'huile comestible ou de mélasse (0 à 20 %).

Les appâts selon ce Brevet EP 208 528 sont obtenus par extrusion à 90-110°C d'un mélange à sec du polymère, de la substance attractive et éventuellement de l'huile comestible.

La Demande Internationale WO 89/12393 décrit des compositions pesticides comprenant de l'EVA, un agent bioactif, une source de protéines/sucres/lipides et éventuellement 0 à 20 % d'huile comestible, une substance attractive, un colorant, un conservateur, un agent répulsif et un biomarqueur.

Les compositions selon cette Demande Internationale PCT WO 89/12393 peuvent être sous forme de blocs ou de comprimés et sont également préparées par extrusion de la composition précitée, soit par fusion du polymère (fusible à une température < 110°C), suivie du mélange de ce dernier avec les autres ingrédients, soit par chauffage, juscu'au point de fusion du polymère, d'un mélange de l'ensemble des ingrédients sous forme sèche.

La Demande de Brevet EP 421 863 décrit des systèmes comprenant deux parties : une enveloppe sous forme tubulaire, obtenue par extrusion et comprenant au moins une substance attractive, au moins une substance agglomérante (polyosides, amidons ou polymères tels que EVA) et éventuellement une substance hydrophobe (huile) et à l'intérieur de la cavité de l'enveloppe, une substance de liaison (mélange de corps gras possédant un point de fusion peu élevé) contenant un principe actif, la substance de liaison épousant la forme interne de l'enveloppe. Une telle enveloppe possède une résistance mécanique et thermique élevée, permettant notamment la distribution par largage aérien.

Toutefois, les différents systèmes obtenus par extrusion présentent un certain nombre d'inconvénients :
- enrobage des particules de substances actives par le polymère (WO 89/12393), qui modifie la vitesse de libération de ces dernières ; en outre, de telles particules alimentaires enrobées sont mal assimilées, en raison d'une mauvaise assimilation des polymères (résistance aux enzymes, en particulier),
- sélection des polymères nécessairement parmi ceux dont le point de fusion est < 110°C (EP 208 528, EP 421 863),
- multiplicité des opérations (EP 421 863 : formation de deux éléments différents, qui sont ensuite reliés ensemble : système hétérogène ou EP 0 240 826),
- texture plus ou moins rigide, qui ne résiste pas au largage aérien et/ou aux conditions extrêmes de température.

Le document US-A-5 169 645 décrit de granules contenant cire.

La présente invention s'est, en conséquence, donné pour but de fournir une composition et un système qui répondent mieux aux besoins de la pratique que les compositions et systèmes de l'Art antérieur, notamment :
. en ce qu'ils sont aisés à réaliser,
. en ce qu'ils sont homogènes,
. en ce qu'ils présentent une texture élastique, particulièrement bien adaptée à une distribution à grande échelle, notamment par largage aérien dans différentes conditions climatiques,
. en ce qu'ils présentent une résistance mécanique et thermique élevées, permettant, selon la ou les substances bioactives à administrer, d'être conservés à des températures comprises entre -30°C et +50°C,
. en ce qu'ils peuvent supporter de telles températures extrêmes sans modification de leur texture et notamment sans suintement lipidique,
. en ce qu'ils sont particulièrement bien adaptés à l'administration orale de substances à effet thérapeutique, diététique, alimentaire, etc..., chez les animaux,
. en ce qu'ils sont attractifs et appétents vis-a-vis de l'animal concerné et présentent donc une texture acceptable par l'animal, par exemple non collante, assurant ainsi une prise effective de la substance bioactive, et
. en ce qu'ils sont stables (forme et dimensions non altérables lors d'un séjour prolongé dans le milieu de distribution), plus particulièrement pour les animaux sauvages ou d'élevage extensif.

La présente invention s'est également donné pour but de fournir un procédé de préparation desdits systèmes et compositions, facile à mettre en oeuvre, peu onéreux et automatisable.

La présente invention a pour objet une composition appétente sous forme solide, du type comprenant au moins un polymère insoluble dans l'eau, une substance attractive et une source lipidique, laquelle composition est caractérisée en ce qu'elle comprend essentiellement :
(a) entre 3 % et 20 % en poids d'au moins un polymère insoluble dans l'eau, choisi parmi les polyamides et les copolymères d'éthylène,
(b) entre 35 et 60 % en poids de substances lipidiques, au moins l'une de ces substances lipidiques étant sous forme solide à température ambiante, le point de fusion de la (les) substance(s) lipidique(s) sous forme solide, étant inférieur à celui du/des polymères, et
(c) entre 5 et 45 % d'au moins une substance appétence et
(d) entre 0 et 50 % d'un autre ingrédient complémentaire convenable et
   en ce qu'elle est susceptible d'être obtenue par:
   (i) fusion des substances lipidiques sous forme solide à une température inférieure à celle du point de fusion du/des polymères, et
   (ii) mélange du/des polymères et des autres composants à la même température qu'en (i).

Selon un mode de réalisation avantageux de ladite composition, elle est susceptible d'être obtenue par:
(i) fusion des substances lipidiques sous forme solide à une température comprise entre 40° et 80°C, et
(ii) mélange du/des polymères et des autres composants dans la même plage de températures.

De manière inattendue, une composition comprenant, en combinaison, au moins un polymère insoluble dans l'eau, au moins une substance lipidique et au moins une substance appétente et obtenue par fusion des substances lipidiques sous forme solide à une température inférieure à la température de fusion des polymères, permet d'obtenir une composition de texture homogène et à consistance de type "pâte d'amande", qui ne modifie pas la vitesse de libération des substances actives qu'elle peut en outre contenir et qui est particulièrement bien adaptée, par ses qualités mécaniques (élasticité, bonne tenue...) et thermiques aux applications recherchées.

Egalement de manière inattendue, malgré la quantité importante de substances lipidiques, on n'observe aucun suintement de lipides entre -30°C et +50°C (températures climatiques extrêmes), du fait de cette texture particulière, dans laquelle le polymère est dispersé, c'est-à-dire en quelque sorte "noyé", ceci étant en particulier dû à la présence de substance appétente.

Le mode particulier de préparation de ladite composition permet, en outre, d'augmenter artificiellement le point de fusion global de la composition, sans en modifier ses qualités d'assimilation.

Le polymère est avantageusement un copolymère éthylène/acétate de vinyle (EVA), comprenant entre 8 et 60 % de groupements acétate de vinyle.

Selon un autre mode de réalisation avantageux de ladite composition, elle comprend jusqu'à 50 % en poids de substance bioactive.

Selon un autre mode de réalisation avantageux de ladite composition, elle comprend optionnellement, au moins l'un des ingrédients complémentaires (d) suivants : des conservateurs, des anti-oxydants, des colorants, des liants classiques tels que amidons, celluloses, cires animales, végétales ou synthétiques, silicones, acides gras à longues chaînes, des traceurs, un ou des édulcorants, des tensio-actifs, des agents de surface et des agents de démoulage.

Le terme "substance lipidique" inclut, mais de manière non limitative, les composés suivants :
a) origine naturelle :
   cire d'abeille, lanoline, saindoux, graisses de porc, de boeuf ou de poisson ; huile de poisson (origine animale) ; cire de Carnauba, les huiles de soja, d'arachide, de colza (origine végétale) ; paraffine, cire microcristalline, vaseline, huiles minérales (origine minérale) ;
b) origine hémi-synthétique ou synthétique :
   alcools gras (alcool cétylique), acides gras (acide stéarique, palmitique), les esters d'alcool et d'acide gras (cire blanche), les huiles végétales polyoxyéthylénées (Labrafils®, Gattefossé), les huiles végétales hydrogénées (Cutina® HR, Henkel), les mono-, di- ou triglycérides d'acides gras (Softisan® 154, Dynamit Nobel).

Le ou les polymères doivent se présenter sous la forme d'une poudre, la plus fine possible, par exemple entre 10 et 400 micromètres.

La ou les substances appétentes peuvent être naturelles ou synthétiques telles que des farines de viandes, ou de poissons, ou des farines végétales, des arômes, etc...

De manière préférée, la composition conforme à l'invention comprend une substance appétente sous forme liquide, arôme liquide de poisson par exemple (à caractère solvant des substances lipidiques) et une substance appétente sous forme solide, telle que farine de viande ou de poisson (à caractère liant).

La présente invention a également pour objet un système pour l'administration orale d'au moins une substance bioactive à des animaux, caractérisé en ce qu'il est constitué d'une composition telle que définie ci-dessus mise sous forme de blocs ou de granules.

Un bloc présente n'importe quelle configuration (cylindrique, parallélépipédique...) et pèse entre 1 et 50 g.

Des granules, de configuration plus ou moins cylindrique ou sphérique pèsent de 0,2 à 2 g.

De manière avantageuse, le système sous forme de bloc inclut la/les substances bioactives dans la composition ci-dessus.

En variante, lesdits blocs englobent ou enveloppent ladite/lesdites substances bioactives ; dans ce cas, ladite substance bioactive n'est pas incluse dans la composition conforme à l'invention, mais se trouve au coeur dudit bloc.

Dans ce dernier cas, la ou les substances bioactives se présentent sous forme solide telle que comprimés, lyophilisats oraux, etc..., ou se présentent sous forme liquide ou pâteuse et peuvent être contenues notamment dans des sachets, gélules, capsules, etc..

La présente invention a également pour objet un procédé de préparation d'une composition conforme à l'invention, caractérisé en ce qu'il comprend :
(i) la fusion des substances lipidiques sous forme solide, à une température inférieure à la température de fusion du/des polymères de ladite composition et
(ii) le mélange du/des polymères et des autres composants à la même température.

Selon un mode de mise en oeuvre avantageux dudit procédé, ladite température est comprise entre 40 et 80°C.

La présente invention a également pour objet un procédé de préparation desdits systèmes, caractérisé en ce qu'il comprend :
(1) la préparation d'une composition conforme à l'invention par :
   (i) fusion des substances lipidiques sous forme solide, à une température inférieure à la température de fusion du/des polymères de ladite composition et
   (ii) mélange du/des polymères et des autres composants à la même température ; et
(2) la mise en forme appropriée des compositions obtenues en (1).

Selon un mode de mise en oeuvre avantageux de ce procédé, ladite température est comprise entre 40 et 80°C.

Selon un autre mode de mise en oeuvre avantageux de ce procédé, l'étape de mise en forme est réalisée par moulage.

Selon un autre mode de mise en oeuvre avantageux de ce procédé, l'étape de mise en forme est réalisée par injection.

Lorsque lesdits systèmes sont mis en forme par moulage ou injection, ils se présentent avantageusement sous la forme de blocs.

Conformément à l'invention, lorsque lesdits systèmes se présentent sous la forme de blocs, la/les substances bioactives peuvent être englobées dans lesdits blocs, comme précisé ci-dessus.

Dans ce cas, (a) une partie de ladite composition conforme à l'invention est répartie dans un moule de conformation appropriée, (b) la/les substances bioactives sont ensuite introduites, puis (c) le reste de ladite composition est introduit dans le moule.

En raison de la texture particulière de la composition conforme à l'invention, un système réalisé de cette manière ne présente pas de fragilité à la jonction des deux parties de la composition.

Selon un autre mode de mise en oeuvre avantageux dudit procédé, l'étape de mise en forme est réalisée par granulation.

De manière avantageuse, lesdits granules sont obtenus par transformation de la composition de texture "pâte d'amande", par passage dans une tour de refroidissement *via* une plaque vibrante d'orifices calibrés pour obtenir directement des granules homogènes de 0,2 à 2 g.

Cette plage de température permet d'envisager l'utilisation des substances actives même sensibles à la chaleur.

Cette température est bien inférieure à la température de fusion du ou des polymères utilisés.

L'addition d'au moins 3 % de polymère(s) et/ou copolymère(s) et d'au moins une substance appétante, dans le mélange chaud, entre 40 et 80°C, et une mise en forme, cela sans nécessiter de transformation classique des polymères habituellement utilisés (fusion), permet, contrairement à ce qui est connu dans l'Art antérieur, d'obtenir de façon inattendue, comme précisé ci-dessus, un système possédant les propriétés suivantes :
- résistance à la chaleur : l'adjonction du ou des polymères augmente considérablement le point de fusion des composants lipidiques en évitant les suintements de ces derniers et la désagrégation du dispositif ;
- élasticité : l'adjonction du ou des polymères, associé à la substance appétente, augmente considérablement la résistance aux chocs ;
- intégrité : entre -30°C et 50°C, le système ne subit aucune dégradation de son aspect, craquelures, division, suintements, etc, aucune perte de ses propriétés physiques ou organoleptiques une fois revenu à une température normale (20-25°C) ;
- attractif et appétent ;
- texture acceptable par l'animal : la transformation, le mélange et la mise en forme à 40-80°C, température bien inférieure à la température de fusion du ou des polymères utilisés, engendrent un aspect et une texture particulièrement adaptés à la prise effective du système par les animaux ;
- possibilité d'avoir toutes les formes et dimensions envisageables selon l'espèce animale cible, selon le moule de conformation utilisé ;
- possibilité d'être broyé par exemple par cryogénie ;
- possibilité, en évitant la répartition, de transformer le mélange homogène à 40-80°C en granules de 0,2 à 2 g, au travers d'une plaque vibrante perforée d'orifices calibrés, reliée à une tour de refroidissement, de 30 cm à 10 m de hauteur, dont l'atmosphère est contrôlée, éventuellement inerte ; cet appareillage assure aux granules une distribution homogène.

Le système conforme à l'invention trouve application notamment en tant que médicament, produit diététique, composition alimentaire ou appât.

Il doit être bien entendu, toutefois, que ces exemples sont donnés uniquement à titre d'illustration de l'objet de l'invention, dont ils ne constituent en aucune manière une limitation.

### EXEMPLE 1 : Complément alimentaire unitaire de 2,5 g pour chiens.

Dans un mélangeur planétaire chauffant, on introduit l'huile de coprah et la paraffine qui sont liquéfiées à 70°C. On introduit sous agitation, en maintenant la température à 70°C environ, tous les autres constituants selon la composition suivante (formule unitaire).

| . substances lipidiques : | |
|---|---|
| huile de coprah | 27,5 % |
| paraffine (50-52°C) | 22,5 % |

| . polymère insoluble : | |
|---|---|
| éthylène/acétate de vinyle* | 10 % |

| . appétent : | |
|---|---|
| farine de viande | 5 % |
| arôme de boeuf | 2 % |

| . principes actifs : | |
|---|---|
| phosphate bicalcique | 20 % |
| vitamines A, D₃, E, B₁, B₂, PP | 6 % |
| amino-acides | 6 % |
| oligo-éléments | 1 % |

| | |
|---|---|
| * avec 28 % d'acétate de vinyle. | |

On homogénéise le mélange pendant 1/2 heure en maintenant la température. La préparation est ensuite répartie dans des alvéoles oblongues en PVC préalablement thermoformées à raison de 2,5 g par alvéole. Les alvéoles pleines sont ensuite refroidies par passage dans un tunnel de congélation (-10°C), et obstruées par sertissage d'un film aluminium.

### EXEMPLE 2 : Tests in vitro et in vivo des systèmes conformes à l'invention obtenus à l'Exemple 1.

a) Tenue à la température :
   les essais ont montré qu'à des températures comprises entre -30°C et +50°C, il n'y avait aucune modification de l'aspect ou de la structure. Ainsi le système, placé sur un papier absorbant conservé à 45°C, se ramollit très légèrement mais ne laisse apparaître aucun suintement, et ne provoque aucune tache de graisse sur le papier absorbant.
   Il n'en est pas de même pour des systèmes dont la composition a été modifiée (remplacement des 10 % d'éthylène/acétate de vinyle par 10 % de paraffine (50-52°C)) : fort ramollissement des systèmes, ils sont graisseux, difficilement manipulables et laissent des taches grasses sur le papier absorbant.
b) Attractivité et appétence :
   les essais ont été réalisés sur trois groupes de six chiens (six petits, six moyens et six grands) répartis en boxes individuels. A la fin du repas, on présente, à chaque chien, deux systèmes dans une gamelle. On note une consommation complète. Le test est renouvelé un quart d'heure après, la consommation est toujours complète.
   Cet essai a été aussi réalisé avec des systèmes conservés en partie à -27°C et en partie à +45°C pendant un mois, puis ramenés à température ambiante pour être présentés aux animaux. On note aucune modification dans la prise et l'absorption du système.
c) Stabilité :
   une étude de stabilité des vitamines a été effectuée sur des échantillons conservés en alvéoles thermoformées, obturées par un film aluminium serti dans une boîte carton à température ambiante (20-25°C) et à la lumière du jour pendant un an. On a noté aucune perte notable de la teneur en vitamines.

### EXAMPLE 3 : Appât vaccinant pour chiens.

Dans un mélangeur planétaire chauffant, on introduit le suif de boeuf et la paraffine qui sont liquéfiés à 55°C. On introduit sous agitation, en maintenant la température à 55°C environ, tous les autres constituants selon la composition suivante (formule unitaire).

| . substances lipidiques : | |
|---|---|
| paraffine (50-52°C) | 20 % |
| suif de boeuf | 30 % |

| . polymère insoluble : | |
|---|---|
| éthylène/acétate de vinyle* | 10 % |

| . appétents : | |
|---|---|
| farine de poisson | 30 % |
| arôme liquide de poisson | 10 % |

| | |
|---|---|
| * avec 28 % d'acétate de vinyle | |

On homogénéise le mélange pendant 1/2 heure en maintenant la température. La préparation est ensuite répartie une première fois dans des moules carrés de 50 mm de côté et 15 mm de profondeur en PVC préalablement thermoformés à raison de 8 g environ par alvéole. Les alvéoles partiellement remplies sont ensuite refroidies jusqu'à environ 30°C et un blister de 25 mm de diamètre et 5 mm d'épaisseur contenant la solution vaccinale est posé sur la première coulée.

On répartit une deuxième fois le mélange à 50°C, pour obtenir un appât de poids final voisin de 31 g, qui immobilise le blister au coeur du dispositif qui se présente alors comme un bloc homogène, et on effectue un passage dans un tunnel de congélation (-30°C) pour refroidir le système mais aussi maintenir l'intégrité du vaccin. Le système obtenu sous forme d'un appât est ensuite démoulé et conservé à -30°C. Il ne présente pas de fragilité à la jonction des deux coulées, car la deuxième coulée est réalisée alors que la première est encore chaude.

### EXEMPLE 4 :

Les tests réalisés dans l'Exemple 2 ont été reproduits avec des systèmes obtenus selon l'Exemple 3.

Les blisters contenus au coeur des systèmes ont été remplis avec une solution colorante de Rhodamine B, utilisée comme marqueur.

Les systèmes avaient été conservés avant l'expérimentation un mois à -27°C. Un système congelé a été placé dans chaque cage ; 24 heures après, on a noté la disparition des systèmes et tous les animaux avaient la gueule colorée.

Ces résultats montrent une bonne attractivité dans les 24 heures qui suivent la distribution et une structure qui favorise la prise. La congélation ne nuit pas à la prise mais permet une conservation optimale des solutions vaccinales.

Des systèmes réalisés selon l'Exemple 3, et des systèmes dont la composition a été modifiée (remplacement des 10 % d'éthylène/acétate de vinyle par 10 % de paraffine (50-52°C)), conservés à 20-25°C et congelés (-27°C) ont été largués par avion à environ 130 mètres d'altitude, au dessus d'une piste cimentée d'aérodrome. Aucun système réalisé selon l'invention n'a présenté de fracture avec séparation entre le blister et le support, et aucun blister n'a été perforé, alors que les systèmes réalisés sans copolymère ont montré de nombreuses détériorations.

### EXEMPLE 5 : Aliment médicamenteux pour poissons sous forme de granules de 0,5 g.

Dans un mélangeur planétaire chauffant, on introduit la paraffine qui est liquéfiée à 55°C.

On introduit sous agitation, en maintenant la température à 55°C environ, tous les autres constituants selon la composition suivante (formule unitaire) :

| . substances lipidiques : | |
|---|---|
| huile de poisson | 10 % |
| paraffine (50-52°C) | 37 % |

| . polymère insoluble : | |
|---|---|
| éthylène/acétate de vinyle* | 17 % |

| . appétent : | |
|---|---|
| farine de poisson | 32 % |

| . principes actifs : | |
|---|---|
| acide oxolinique | 0,5 % |
| vitamines A, D₃, E, B₁, B₂, B₆ | 2,5 % |
| oligo-éléments | 1 % |

| | |
|---|---|
| * avec 28 % d'acétate de vinyle | |

On homogénéise le mélange pendant 1/2 heure en maintenant la température. On transforme le mélange homogène chaud en granules de 0,5 g, au travers d'une plaque vibrante perforée d'orifices calibrés, reliée à une tour de refroidissement, de 3 m de hauteur, dont l'atmosphère est maintenue à 0°C environ.

## Revendications

1. Composition appétente sous forme solide, du type comprenant au moins un polymère insoluble dans l'eau, une substance attractive et une source lipidique, laquelle composition est caractérisée en ce qu'elle comprend essentiellement :
(a) entre 3 % et 20 % en poids d'au moins un polymère insoluble dans l'eau, choisi parmi les polyamides et les copolymères d'éthylène,
(b) entre 35 et 60 % en poids de substances lipidiques, au moins l'une de ces substances lipidiques étant sous forme solide à température ambiante, le point de fusion de la (les) substance(s) lipidique(s) sous forme solide, étant inférieur à celui du/des polymères, et
(c) entre 5 et 45 % d'au moins une substance appétente et
(d) entre 0 et 50 % d'un autre ingrédient complémentaire convenable et
en ce qu'elle est susceptible d'être obtenue par:
(i) fusion des substances lipidiques sous forme solide à une température inférieure à celle du point de fusion du/des polymères, et
(ii) mélange du/des polymères et des autres composants à la même temperature qu'en (i).

2. Composition selon la revendication 1, caractérisée en ce qu'elle est susceptible d'être obtenue par:
(i) fusion des substances lipidiques sous forme solide à une température comprise entre 40° et 80°C, et
(ii) mélange du/des polymères et des autres composants dans la même plage de températures.

3. Composition selon la revendication 1 ou la revendication 2, caractérisée en ce qu'elle comprend jusqu'à 50 % en poids de substance bioactive.

4. Composition selon l'une quelconque des revendications 1 à 3, caractérisée en ce qu'elle comprend optionnellement, au moins l'un des ingrédients complémentaires (d) suivants : des conservateurs, des anti-oxydants, des colorants, des liants, des traceurs, un ou des édulcorants, des tensio-actifs, des agents de surface et des agents de démoulage.

5. Composition selon l'une quelconque des revendications 1 à 4, caractérisée en ce que la substance appétente est sélectionnée parmi les arômes liquides et les farines.

6. Système pour l'administration orale d'au moins une substance bioactive à des animaux, caractérisé en ce qu'il est constitué par une composition selon l'une quelconque des revendications 1 à 5, mise sous forme de blocs.

7. Système pour l'administration orale d'au moins une substance bioactive à des animaux, caractérisé en ce qu'il est constitué par une composition selon l'une quelconque des revendications 1 à 5, mise sous forme de granules.

8. Système selon la revendication 6, caractérisé en ce que la/les substances bioactives sont incluses dans la composition constituant lesdits blocs.

9. Système selon la revendication 6, caractérisé en ce que la/les substances bioactives sont englobées dans lesdits blocs.

10. Procédé de préparation d'une composition selon l'une quelconque des revendications 1 à 5, caractérisé en ce qu'il comprend :
(i) la fusion des substances lipidiques sous forme solide, à une température inférieure à la température de fusion du/des polymères de ladite composition et
(ii) le mélange du/des polymères et des autres composants à la même température.

11. Procédé selon la revendication 10, caractérisé en ce que ladite température est comprise entre 40 et 80°C.

12. Procédé de préparation des systèmes selon l'une quelconque des revendications 6 à 9, caractérisé en ce qu'il comprend :
(1) la préparation d'une composition selon l'une quelconque des revendications 1 à 5 par :
(i) fusion des substances lipidiques sous une forme solide, à une température inférieure à la température de fusion du/des polymères de ladite composition et
(ii) mélange du/des polymères et des autres composants à la même température ; et
(2) la mise en forme appropriée des compositions obtenues en (1).

13. Procédé selon la revendication 12, caractérisé en ce que ladite température est comprise entre 40 et 80°C.

14. Procédé selon la revendication 12 ou la revendication 13, caractérisé en ce que l'étape de mise en forme est réalisée par moulage.

15. Procédé selon la revendication 12 ou la revendication 13, caractérisé en ce que l'étape de mise en forme est réalisée par injection.

16. Procédé selon la revendication 14 ou la revendication 15, caractérisé en ce que lesdits systèmes se présentent sous forme de blocs.

17. Procédé selon la revendication 12 ou la revendication 13, caractérisé en ce que l'étape de mise en forme est réalisée par granulation.

18. Médicament, caractérisé en ce qu'il est constitué par un système selon l'une quelconque des revendications 6 à 17.

19. Produit diététique, caractérisé en ce qu'il est constitué par un système selon l'une quelconque des revendications 6 à 17.

20. Produit alimentaire, caractérisé en ce qu'il est constitué par un système selon l'une quelconque des revendications 6 à 17.

21. Appât, caractérisé en ce qu'il est constitué par un système selon l'une quelconque des revendications 6 à 17.

## Claims

1. Palatable composition in solid form, of the type comprising at least one water-insoluble polymer, an attractive substance and lipid source, which composition is characterized in that it comprises essentially:
(a) between 3% and 20% by weight of at least one water-insoluble polymer chosen from polyamides and ethylene copolymers,
(b) between 35 and 60% by weight of lipid substances, at least one of these lipid substances being in solid form at room temperature, the melting point of the lipid substance(s) in lipid form being less than that of the polymer(s), and
(c) between 5 and 45% of at least one palatable substance and
(d) between 0 and 50% of another suitable additional ingredient and
in that it can be obtained by:
(i) melting of the lipid substances in liquid form at a temperature less than that of the melting point of the polymer(s), and
(ii) mixing of the polymer(s) and the other constituents at the same temperature as in (i).

2. Composition according to Claim 1, characterized in that it can be obtained by:
(i) melting of the lipid substances in solid form at a temperature of between 40° and 80°C, and
(ii) mixing of the polymer(s) and of the other constituents in the same temperature range.

3. Composition according to Claim 1 or Claim 2, characterized in that it comprises up to 50% by weight of bioactive substance.

4. Composition according to any one of Claims 1 to 3, characterized in that it optionally comprises at least one of the following additional ingredients (d): preservatives, antioxidants, colorants, binders, tracers, one or more sweeteners, surfactants, surface-active agents and unmoulding agents.

5. Composition according to any one of Claims 1 to 4, characterized in that the palatable substance is selected from liquid flavourings and meals.

6. System for the oral administration of at least one bioactive substance to animals, characterized in that it consists of a composition according to any one of Claims 1 to 5, made in the form of blocks.

7. System for the oral administration of at least one bioactive substance to animals, characterized in that it consists of a composition according to any one of Claims 1 to 5, made in the form of granules.

8. System according to Claim 6, characterized in that the bioactive substance(s) are included in the composition constituting the said blocks.

9. System according to Claim 6, characterized in that the bioactive substance(s) are enclosed in the said blocks.

10. Method for preparing a composition according to any one of Claims 1 to 5, characterized in that it comprises:
(i) the melting of the lipid substances in solid form, at a temperature less than the melting temperature of the polymer(s) of the said composition and
(ii) the mixing of the polymer(s) and of the other constituents at the same temperature.

11. Method according to Claim 10, characterized in that the said temperature is between 40 and 80°C.

12. Method for preparing the systems according to any one of Claims 6 to 9, characterized in that it comprises:
(1) the preparation of a composition according to any one of Claims 1 to 5 by:
(i) melting the lipid substances in solid form, at a temperature less than the melting temperature of the polymer(s) of the said composition and
(ii) mixing the polymer(s) and the other constituents at the same temperature; and
(2) the appropriate shaping of the compositions obtained in (1).

13. Method according to Claim 12, characterized in that the said temperature is between 40 and 80°C.

14. Method according to Claim 12 or Claim 13, characterized in that the shaping stage is performed by moulding.

15. Method according to Claim 12 or Claim 13, characterized in that the shaping stage is performed by injection.

16. Method according to Claim 14 or Claim 15, characterized in that the said systems are provided in the form of blocks.

17. Method according to Claim 12 or Claim 13, characterized in that the shaping stage is performed by granulation.

18. Medicament, characterized in that it consists of a system according to any one of Claims 6 to 17.

19. Dietetic product, characterized in that it consists of a system according to any one of Claims 6 to 17.

20. Food product, characterized in that it consists of a system according to any one of Claims 6 to 17.

21. Bait, characterized in that it consists of a system according to any one of Claims 6 to 17.

## Patentansprüche

1. Appetitanregende Zusammensetzung in fester Form des Typs, umfassend mindestens ein in Wasser unlösliches Polymeres, eine anziehende Substanz und eine Lipidquelle, dadurch **gekennzeichnet,** daß sie im wesentlichen umfaßt
(a) zwischen 3 und 20 Gew.-% mindestens eines in Wasser unlöslichen Polymeren, ausgewählt aus Polyamiden und Ethylencopolymeren,
(b) zwischen 35 und 60 Gew.-% Lipidsubstanzen, wobei mindestens eine dieser Lipidsubstanzen in fester Form bei Umgebungstemperatur vorliegt, wobei der Schmelzpunkt der Lipidsubstanz(en) in fester Form unter dem des/der Polymeren liegt und
(c) zwischen 5 und 45 % mindestens einer appetitanregenden Substanz und
(d) zwischen 0 und 50 % eines geeigneten anderen komplementären Inhaltsstoffs, und daß sie erhalten werden kann durch
(i) Schmelzen der Lipidsubstanzen in fester Form, bei einer Temperatur unter dem Schmelzpunkt des/der Polymeren und
(ii) Vermischen des/der Polymeren und der anderen Bestandteile bei der gleichen Temperatur wie in (i).

2. Zusammensetzung nach Anspruch 1, dadurch **gekennzeichnet,** daß sie erhalten werden kann durch
(i) Schmelzen der Lipidsubstanzen in fester Form bei einer Temperatur zwischen 40 und 80°C und
(ii) Vermischen des/der Polymeren und der anderen Bestandteile im gleichen Temperaturbereich.

3. Zusammensetzung nach Anspruch 1 oder 2, dadurch **gekennzeichnet,** daß sie bis zu 50 Gew.-% einer bioaktiven Substanz umfaßt.

4. Zusammensetzung nach einem der Ansprüche 1 bis 3, dadurch **gekennzeichnet,** daß sie gegebenenfalls mindestens einen der folgenden komplementären Inhaltsstoffe (d) umfaßt: Konservierungsstoffe, Antioxidantien, Farbstoffe, Bindemittel, Tracer, Süßstoff(e), grenzflächenaktive Mittel, oberflächenaktive Mittel und Entformungsmittel.

5. Zusammensetzung nach einem der Ansprüche 1 bis 4, dadurch **gekennzeichnet,** daß die appetitanregende Substanz aus flüssigen Geschmackstoffen und Mehlen ausgewählt ist.

6. System zur oralen Verabreichung mindestens einer bioaktiven Substanz an Tiere, dadurch **gekennzeichnet,** daß es aus einer Zusammensetzung nach einem der Ansprüche 1 bis 5 in Blockform besteht.

7. System zur oralen Verabreichung mindestens einer bioaktiven Substanz an Tiere, dadurch **gekennzeichnet,** daß es aus einer Zusammensetzung nach einem der Ansprüche 1 bis 5 in Granulatform besteht.

8. System nach Anspruch 6, dadurch **gekennzeichnet,** daß die bioaktive Substanz(en) in der die Blöcke bildenden Zusammensetzung eingeschlossen ist/sind.

9. System nach Anspruch 6, dadurch **gekennzeichnet,** daß die bioaktive Substanz(en) in den Blöcken englobiert ist/sind.

10. Verfahren zur Herstellung einer Zusammensetzung nach einem der Ansprüche 1 bis 5, dadurch **gekennzeichnet,** daß es umfaßt
(i) das Schmelzen der Lipidsubstanzen in fester Form bei einer Temperatur unter der Schmelztemperatur des/der Polymeren der Zusammensetzung und
(ii) Vermischen des/der Polymeren und anderer Bestandteile bei der gleichen Temperatur.

11. Verfahren nach Anspruch 10, dadurch **gekennzeichnet,** daß die Temperatur zwischen 40 und 80°C liegt.

12. Verfahren zur Herstellung von Systemen nach einem der Ansprüche 6 bis 9, dadurch **gekennzeichnet,** daß es umfaßt:
(1) die Herstellung einer Zusammensetzung nach einem der Ansprüche 1 bis 5 durch
(i) Schmelzen der Lipidsubstanzen in fester Form bei einer Temperatur unter der Schmelztemperatur des/der Polymeren der Zusammensetzung und
(ii) Vermischen des/der Polymeren und anderer Bestandteile bei der gleichen Temperatur und
(2) Formgebung der in (1) erhaltenen Zusammensetzungen in geeigneter Weise.

13. Verfahren nach Anspruch 12, dadurch **gekennzeichnet,** daß die Temperatur zwischen 40 und 80°C liegt.

14. Verfahren nach Anspruch 12 oder 13, dadurch **gekennzeichnet,** daß die Stufe der Formgebung durch Formgießen durchgeführt wird.

15. Verfahren nach Anspruch 12 oder 13, dadurch **gekennzeichnet,** daß die Stufe der Formgebung durch Injektion durchgeführt wird.

16. Verfahren nach Anspruch 14 oder 15, dadurch **gekennzeichnet,** daß die Systeme in Blockform vorliegen.

17. Verfahren nach Anspruch 12 oder 13, dadurch **gekennzeichnet,** daß die Stufe der Formgebung durch Granulation durchgeführt wird.

18. Medikament, dadurch **gekennzeichnet,** daß es aus einem System nach einem der Ansprüche 6 bis 17 besteht.

19. Diätetisches Produkt, dadurch **gekennzeichnet,** daß es aus einem System nach einem der Ansprüche 6 bis 17 besteht.

20. Lebensmittelprodukt, dadurch **gekennzeichnet,** daß es aus einem System nach einem der Ansprüche 6 bis 17 besteht.

21. Lockmittel, dadurch **gekennzeichnet,** daß es aus einem System nach einem der Ansprüche 6 bis 17 besteht.
